Europäisches Patentamt

European Patent Office

Office européen des brevets

(11) Publication number: **0 162 457**
**B1**

## (12) EUROPEAN PATENT SPECIFICATION

(45) Date of publication of patent specification: **26.07.89**

(51) Int. Cl.⁴: **C 07 C 17/34,** C 07 C 21/06, B 01 J 23/10

(21) Application number: **85106285.1**

(22) Date of filing: **22.05.85**

(54) Catalytic dehydrohalogenation process.

(30) Priority: **24.05.84 US 613551**

(43) Date of publication of application:
**27.11.85 Bulletin 85/48**

(45) Publication of the grant of the patent:
**26.07.89 Bulletin 89/30**

(84) Designated Contracting States:
**BE DE FR GB IT NL SE**

(56) References cited:
US-A-3 927 131

PATENT ABSTRACTS OF JAPAN, vol. 4, no. 190
(C-37)672r, 26th December 1980; & JP - A - 55
130 923 (MITSUBISHI JUKOGYO K.K.) 11-10-
1980

SOVIET INVENTIONS ILLUSTRATED, Section
Chemical, Week D29, 26th August 1981,
abstract no. A41 E16, Derwent Publications
Ltd., London, GB; & SU - A - 775 101 (SARAT
UNIV CHEM INS) 30-10-1980

SOVIET INVENTIONS ILLUSTRATED, Section
Chemical, Week K09, 13th April 1983, abstract
no. J04 E16, Derwent Publications Ltd.,
London, GB; & SU - A - 923 587 (SARAT CHEM
INST) 30-04-1982

(73) Proprietor: **The B.F. GOODRICH Company
Dept. 0015 WHB-6 500 South Main Street
Akron, Ohio 44318 (US)**

(72) Inventor: **Magistro, Angelo Joseph
11017 Brook View Drive
Brecksville Ohio 44141 (US)**

(74) Representative: **von Kreisler, Alek, Dipl.-Chem.
et al
Deichmannhaus am Hauptbahnhof
D-5000 Köln 1 (DE)**

(56) References cited:
CHEMICAL ABSTRACTS, vol. 97, no. 25, 20th
December 1982, page 789, column 1, abstract
no. 215442j, Columbus, Ohio, US; YU.N. USOV
et al.: "C atalytic activity of rare earth
itaconates during dehydrohalogenation of
chloroolefins", & KINET. KATAL. 1982, 23(4),
1017

Courier Press, Leamington Spa, England.

## Description

Background of the invention

It is well known that vinyl chloride monomer (VCM) can be produced from 1,2-dichloroethane (commercially called ethylene dichloride or EDC) by splitting off a molecule of hydrogen chloride. The dehydrohalogenation reaction (commonly called "cracking" in the trade) is typically accomplished by heating the EDC, in the absence of a catalyst, in an inert atmosphere using high temperature and pressure; usually about 550°C and at a pressure of about 300 to 600 psi (21—42 kg/cm$^2$; 2,070,000 to 4,140,000 Pa).

A serious disadvantage of the standard pyrolysis or thermal process is the need of a large amount of costly heat energy to accomplish the cracking. Another serious difficulty encountered in such a process is the rapid deposition of solid by-products (coke) on the tubes which limits the process to relatively short operating periods before cleaning of the tubes is required. Lastly, in these high temperature processes, undesirable side reactions frequently occur which lower the conversion of EDC.

To reduce the problems observed in the standard pyrolytic cracking process various catalytic processes have been proposed. For example, U.S. Patent No. 2,877,277 teaches the use of an alkaline earth metal hydroxide in aqueous solution to promote the dehydrochlorination of EDC to VCM, and refers to the prior use of alkali metal hydroxides. These processes suffer from the disadvantages of a very long contact time (30 minutes to 2.5 hours) and difficulty in the separation and purification of the resultant VCM from the aqueous solution.

U.S. Patent No. 3,290,399 teaches the use of graphite to promote the cracking reaction, and references the use of activated carbon washed with nitric acid as disclosed in British Patent No. 823,285. The processes suffer in that reaction temperatures and pressures are still high, up to 600°C.

Japanese Patent No. 24870/64 teaches the use of a catalyst of a boron compound such as boric acid on activated charcoal.

Lastly, U.S. Patent No. 3,896,182 teaches that the clogging of pyrolysis reactor tubes by carbonaceous substances (coking) can be reduced by conducting the reaction in the substantial absence of oxygen, below 2.0 ppm of oxygen present.

While such processes may be commercially feasible, there has been and still is a continuing search for catalytic materials which will improve the dehydrohalogenation process by enabling the use of lower operating temperatures and pressures and further reduction in undesirable side reactions and coking.

Summary of the invention

It has been unexpectedly found that conversion of EDC to VCM with less energy expenditure and reduced coking can be obtained by a process which comprises dehydrohalogenating the EDC to VCM, in the presence of oxygen, employing, as a catalyst, a composition comprising a rare earth metal chloride on a zeolite. Using the rare earth metal chloride-zeolite catalysts of the instant invention, the cracking process can be operated at a temperature as low as 150°C up to 500°C, and more preferably from about 200°C to about 350°C, at pressures from atmospheric to about 300 psi (1—21 kg/cm$^2$; 101,400—2,070,000 Pa).

Detailed description

Vinyl chloride monomer (VCM) can be prepared by various processes, but the most common process involves the formation of EDC by the reaction between ethylene and chloride or hydrogen chloride, followed by a dehydrohalogenation step wherein the EDC is thermally cracked under pressure to VCM and by-product hydrogen chloride. The hydrogen chloride is typically recovered and used in an oxyhydrochlorination step wherein the hydrogen chloride is reacted with additional ethylene to produce EDC which is, in turn, fed to the cracking step. The VCM is recovered, purified, and used extensively as a monomer in the manufacture of polyvinyl chloride (PVC) resins, a large volume, versatile plastic material.

In the typical cracking process, large amounts of natural gas are burned to supply the heat energy to operate the cracking furnaces. In these processes, the EDC is vaporized and passed through a tube-type furnace which is directly heated to a temperature in the range of about 500°C to about 600°C. In the present, standard commercial cracking process no solid cracking catalyst is employed. In the absence of a catalyst, the reaction is a thermally induced dehydrochlorination whereby one molecule of hydrogen chloride is solit out of each EDC molecule, resulting in one molecule of VCM.

The present invention encompasses the use of many process design configurations such as a single fluid-bed with reactants fed into the reaction zone through a distributor plate, fluid cat cracking technology where the oxygen is fed in at a separate regeneration zone in the reactor, a fixed bed cracking furnace, or any other known designs. The heat energy can be supplied in any known manner, but typically is supplied by burning natural gas. The EDC is vaporized and passed through the fluid bed or tubes in the furnace where the catalyst is present. The ratio of oxygen to EDC in the feed stream is controlled to accomplish the objectives of this invention and yet operate safely.

The temperatures employed in the present process range from as low as 150°C up to about 500°C, more preferably from about 200°C to about 350°C, and most preferably from about 250°C to about 325°C. In the process, pressures will range from about one atmosphere (1 kg/cm$^2$; 101,400 Pa) up to twenty (20) (21 kg/cm$^2$; 2,070,000 Pa) atmospheres, but more typically range from about five (5) to about fifteen (15) atmospheres (5—11 kg/cm$^2$; 507,000—1,520,000 Pa).

A critical feature of the present invention comprises the use of an improved catalyst for the cracking step. Specifically, the catalyst is comprised of a rare earth metal chloride deposited on a zeolite. Both natural and synthetic zeolites can be used in the present invention. Examples of natural zeolites are analcite, erionite, chabazite, faujasite, mordenite, gmelinite, mazzite, offertite, heulandite, natrolite, stilbite, and thomsonite.

The synthetic zeolites can be made in a variety of forms ranging from gelatinous to porous and sandlike. Examples of known synthetic zeolites are Type A (described in U.S. Patent No. 2,882,243); Type X (described in U.S. Patent No. 2,882,244); Type Y (described in U.S. Patent No. 3,130,007); Type R (described in U.S. Patent No. 3,030,181); and other zeolites as described in the December 13, 1982 article in "Chemical and Engineering News" entitled "Shape Selectivity Key to Designed Catalysts" by Mr. Joseph Hoggin. For the purposes of this invention, the solid zeolites are preferred, and particularly those synthetic solid zeolites having pore openings of 0,5 nm (5 Å) or more. Synthetic zeolites having a pore opening of over 0,5 nm (5 Å) are readily available commercially from Englehard Minerals and Chemicals and Linde Division of Union Carbide Corporation. Examples of such commercially-available zeolites are Englehard's HFZ-20, HFZ-33, and HFZ-55.

The rare earth metal chloride can be the chloride of any rare earth metal as found in the Periodic Table numbers 57 (Lanthanum) through 71 (Lutetium). The rare earth metals Lanthanum (La), Praeseodymium (Pr), Neodymium (Nd), and the rare earth metal mixture Dydinium which consists essentially of La, Nd, Pr, Samarium (Sm) and Cerium (Ce), are all readily available and, hence, are preferred rare earth metals. The most preferred rare earth metal is Lanthanum, and the most preferred rare earth metal chloride is Lanthanum Chloride ($LaCl_3$).

The amount of rare earth metal chloride employed with the zeolite can be from about 0.1% up to 30% by weight of the total weight of the catalyst. More preferably, the weight of the rare earth metal chloride is from about 2% to about 20%, and most preferably from about 4% to about 16% by weight. The optimum level of rare earth metal chloride on the zeolite may vary with the type of zeolite employed. The catalysts of this invention can also contain levels of other metals which are often used to impart stability and the like. Examples of such optional materials are alkali and alkaline earth metal oxides and chlorides such as Lithium and Potassium Oxide and Chloride, Magnesium Chloride, and Strontium Chloride. These additional metals can be employed in up to 5% by weight based on the total weight of the catalyst.

The rare earth metal chloride(s), and other optional metal chlorides and oxides, can be deposited on the zeolite in any known manner. Preferably, an aqueous solution(s) of the metal(s) is prepared and contacted with the zeolite, which mixture is then filtered and dried at temperatures below about 200°C, followed by calcination at a temperature from about 500°C to 1000°C for about 2 to 16 hours. A calcination temperature of about 500°C to about 800°C is preferred. Calcining the catalyst mixture at high temperatures, i.e. above 800°C, can reduce the stability and/or effectiveness of the resultant catalyst. The concentration of the metal(s) in the aqueous solution(s) is determined by the desired level of metal on the final catalyst. If more than one metal is added to the zeolite, this can be done simultaneously or sequentially by adding each metal after each drying step. It is possible to add part or all of the metal(s) to the zeolite while the zeolite is in the reactor area, and also to add additional metal(s) to the zeolite during operation to replenish metal(s) lost during the reaction.

The zeolite chosen should have a form or particle size permitting it to be conveniently placed or packed in the cracking reactor. The rare earth metal chloride-zeolite catalyst is placed in the cracking reactor in such fashion as to allow the passage of vapor therethrough. The catalyst may be fixed in the reactor bed or used as a fluidized bed. Typically, the cracking reactor is purged with nitrogen before the EDC and oxygen are introduced into the reactor. When the EDC comes in contact with the catalyst the dehydrochlorination reaction or cracking proceeds smoothly, rapidly converting the EDC to VCM and by-product HCl.

The amount of oxygen employed in the process, either as pure oxygen or air, is from about 0.05 mole to about 0.5 mole of oxygen per mole of EDC. Below 0.05 mole, increased carbon buildup is observed, and above 0.5 mole, increased formation of carbon oxides is observed. The preferred molar ratio of oxygen to EDC employed is from about 0.15 mole to 0.3 mole per mole of EDC. Not all the oxygen need be added in the reaction zone; i.e. the oxygen can be staged or added in a separate zone. The oxygen is added to the reactor in a manner and flow to ensure safety and operations under non-explosive conditions.

While the cracking reaction may be operated at atmospheric pressure, it is preferred in the present invention to operate at superatmospheric pressures of from about 5 to 20 atmospheres (5—11 kg/cm²; 507,000—1,520,000 Pa). Generally, at higher pressures, less coking and less formation of undesirable chlorohydrocarbon by-products, such as $CCl_4$, occurs than when operating at atmospheric pressure. If necessary, the reactor can be shut down and the carbon formation, if any, can be readily removed by conventional means; i.e. heating the reactor at elevated temperatures usually in the range of over 300°C to about 700°C. Also, the carbon can be removed and the catalyst regenerated in a continuous manner using a fluid cat cracking reactor design as shown in the "Chemical and Engineering News" article previously referenced.

The reaction or contact time of the EDC with the catalyst in the reactor can be varied depending on the volume and shape of the reactor and the flow rate of EDC and oxygen. The contact time necessary between the EDC and catalyst to promote the desired dehydrochlorination reaction is obtained by controlling the space velocity of the gaseous material passing through the reaction zone. It has been found, however, that for most reactors a contact time as high as about 60 seconds and as low as 0.5 second can be employed. If the contact time is too low the quantity of unreacted EDC is too high. On the other hand, if the contact time is too high, the amount of carbon oxide and by-product impurities increase. One can readily adjust the gaseous feed rate to obtain the optimum contact time for any particular type reactor. Contact times in the experiments detailed in the following Examples ranged from about 5 seconds to about 30 seconds.

The gaseous mixture withdrawn from the reaction zone can be passed directly to a condenser to recover the condensable materials including the EDC, and hydrogen chloride passed overhead to be collected, and then often recycled to an oxyhydrochlorination process to be used in making more EDC. Alternatively, the gases leaving the reaction zone can be cooled and subjected to fractional distillation under pressure, preferably at lower pressure than that used for the cracking. The hydrogen chloride separates first and is recovered or recycled. The uncondensed gases can be vented into a separate recovery or treatment zone wherein the VCM and unreacted EDC are recovered and fractionally distilled to remove the VCM. The unreacted EDC is recycled to the cracking zone.

Present commercial cracking processes typically operate at above 500°C to 600°C, at pressures of 300 to 600 psi (21—42 kg/cm$^2$; 2,070,000—4,140,000 Pa), and have contact times of about 1 to 5 seconds. For example, operating with no catalyst (thermal cracking only), at a temperature of 560°C with a one second contact time, an EDC conversion of up to 80% can be obtained. At 530°C, the EDC conversion drops to about 50%, and at 500°C, the EDC conversion drops to about 20%. Operation below 500°C is not economically feasible. Hence, the standard thermal EDC cracking processes require high temperature operation and very high energy use. Further, these processes typically suffer from high coking which necessitates shut down and decoking about once each month.

In contrast, the process of this invention results in effective cracking of EDC to VCM with significant energy savings and little coke formation, thereby permitting long operation between shut downs. The formation of carbon oxides and by-product chlorinated hydrocarbon can be as low as under 10%. A small amount, up to 5% and usually no more than 2%, of ethylene may be produced.

The following Examples are given to more specifically define the present invention. In the examples, all parts and percents are by weight, unless otherwise indicated.

Example of preparation of catalyst

500 grams of HEZ-55, a synthetic zeolite obtained from Englehard Minerals and Chemicals, was added to an aqueous solution of 5.77 grams of Lanthanum chloride, (LaCl$_3$ · 6H$_2$O) in 40 cc of distilled water, and stirred to obtain uniform mixing. The mixture of LaCl$_3$ on HEZ-55 was filtered out of solution, dried at 110°C for 16 hours, followed by calcination at 500°C for 16 hours. The rare earth metal chloride-zeolite catalyst contained eight percent (8%) by weight of La metal as analyzed by X-ray fluorescence.

The procedure was essentially repeated using HFZ-20 zeolite and HFZ-33 zeolite in place of HEZ-55 zeolite, and varying the amount of LaCl$_3$ impregnated onto the zeolites. If and when additional, optional metals were added, such as lithium, potassium, magnesium, or strontium, these metals were also added as metal chloride salts in aqueous solution and were added simultaneously with the LaCl$_3$. Conditions of drying and calcination were essentially the same in all catalyst preparations, except as expressly stated below.

Examples of the catalysts of the invention, prepared as described above, are: 8% LaCl$_3$ on HFZ-33; 8% LaCl$_3$, 4% LiCl on HFZ-33; 16% LaCl$_3$ on HFZ-33; 16% LaCl$_3$ on HFZ-55; 8% LaCl$_3$, 4% KCl on HFZ-55, 8% LaCl$_3$, 4% MgCl$_2$ on HFZ-20; and 8% LaCl$_3$, 4% SrCl$_2$ on HFZ-20.

Experiments

A series of experiments were conducted using a laboratory reactor comprising a 30 mm diameter by 45 cm long glass reactor made of pyrex. The catalyst was placed in the reactor and the process was conducted as a one-pass, continuous fluid bed catalytic process. The reactor was first purged with nitrogen gas, followed by introduction of EDC and oxygen (as air). The exit gases were collected and periodic samples were analyzed for EDC, VCM, HCl and other products using a gas chromatograph, Model No. 810 made by Hewlett-Packard and using DC-200 as a packing material. The following examples show data that was obtained using the above-described apparatus and at the oxygen/EDC molar ratios, temperatures, and contact times as indicated, operating at a pressure of about 30 psi (2 kg/cm$^2$; 1,000,000 Pa). Mol % conversion of EDC was determined using the formula

$$\frac{1\text{-millimoles EDC out}}{\text{millimoles EDC feed in}} \times 100$$

and % yield of VCM, carbon oxides, and other products were determined as using the formula

$$\frac{\text{millimoles product out}}{\text{EDC conversion}} \times 100$$

4

## Example 1

This example compares the use of zeolite alone (Z) as a catalyst versus the use of the following catalysts of the invention; (A) a catalyst of 8% $LaCl_3$ on zeolite (B), 8% $LaCl_3$, 4% LiCl on zeolite; and (C) 16% $LaCl_3$ on zeolite. The zeolite used was HFZ-33.

| Run | $O_2$/EDC ratio | Contact time (sec) | Temperature (°C) | Mole % conv. EDC | % Yield | |
|-----|-----|-----|-----|-----|-----|-----|
| | | | | | VCM | $CO+CO_2$ |
| Z1 | 0.26 | 17.3 | 250 | 12.9 | 61.7 | 31.1 |
| 2 | 0.26 | 15.8 | 300 | 23.5 | 58.4 | 38.7 |
| 3 | 0.21 | 11.5 | 300 | 13.5 | 61.9 | 38.1 |
| A1 | 0.29 | 13.9 | 300 | 51.8 | 78.1 | 20.7 |
| 2 | 0.20 | 10.9 | 300 | 35.8 | 90.1 | 9.2 |
| 3 | 0.20 | 15.7 | 300 | 35.7 | 83.2 | 15.5 |
| B1 | 0.29 | 15.2 | 250 | 32.8 | 48.1 | 33.3 |
| 2 | 0.20 | 11.9 | 250 | 14.5 | 60.0 | 20.3 |
| 3 | 0.20 | 10.9 | 300 | 41.3 | 68.7 | 21.9 |
| C1 | 0.29 | 15.2 | 250 | 10.6 | 64.1 | 33.4 |
| 2 | 0.29 | 13.9 | 300 | 24.9 | 57.0 | 41.4 |
| 3 | 0.20 | 10.9 | 300 | 12.5 | 59.9 | 38.2 |

As can be seen from the data, the use of a catalyst of a rare earth metal chloride on zeolite results in significantly higher EDC conversion. The level of 16% $LaCl_3$ has exceeded the optimum concentration on the HFZ-33 zeolite. Example 5 shows a level of 16% $LaCl_3$ on HFZ-55.

## Example 2

The following data is from runs using a catalyst of (A) 8% $LaCl_3$ on HFZ-33 and (B) 8% $LaCl_3$, 4% LiCl on HFZ-33.

| Run | $O_2$/EDC ratio | Contact time (sec) | Temperature (°C) | Mole % conv. EDC | % Yield | |
|-----|-----|-----|-----|-----|-----|-----|
| | | | | | VCM | $CO+CO_2$ |
| A | | | | | | |
| 1 | 0.29 | 5.2 | 250 | 16.6 | 91.1 | 9.1 |
| 2 | 0.29 | 4.8 | 300 | 37.7 | 86.9 | 13.1 |
| 3 | 0.20 | 10.9 | 300 | 35.8 | 90.1 | 9.2 |
| 4 | 0.20 | 15.7 | 300 | 38.9 | 83.3 | 15.4 |
| B | | | | | | |
| 1 | 0.29 | 15.2 | 250 | 32.8 | 48.1 | 34.3 |
| 2 | 0.29 | 13.9 | 300 | 50.2 | 59.6 | 33.0 |
| 3 | 0.20 | 10.9 | 300 | 41.3 | 68.7 | 21.9 |
| 4 | 0.20 | 15.7 | 300 | 42.2 | 65.4 | 24.8 |

## Example 3

The example shows the use of catalysts of (A) 8% $LaCl_3$, 4% $MgCl_2$ on HFZ-20 and (B) 8% $LaCl_3$, 4% $SrCl_2$ on HFZ-20.

| Run | $O_2/EDC$ ratio | Contact time (sec) | Tempera-ture (°C) | Mole % conv. EDC | % Yield | |
|---|---|---|---|---|---|---|
| | | | | | VCM | $CO+CO_2$ |
| A | | | | | | |
| 1 | 0.29 | 15.2 | 250 | 28.8 | 46.8 | 41.6 |
| 2 | 0.20 | 5.8 | 300 | 12.0 | 60.4 | 30.3 |
| 3 | 0.20 | 10.9 | 300 | 25.4 | 69.0 | 29.9 |
| 4 | 0.20 | 26.1 | 300 | 63.9 | 60.0 | 30.4 |
| B | | | | | | |
| 1 | 0.29 | 15.2 | 250 | 35.1 | 36.7 | 57.7 |
| 2 | 0.20 | 17.2 | 250 | 11.7 | 65.0 | 30.7 |
| 3 | 0.29 | 13.9 | 300 | 31.3 | 52.5 | 44.6 |
| 4 | 0.20 | 15.7 | 300 | 25.3 | 54.1 | 42.6 |
| 5 | 0.20 | 10.9 | 300 | 20.5 | 63.4 | 33.2 |
| 6 | 0.20 | 6.9 | 300 | 17.7 | 73.0 | 26.0 |

Example 4

The data shows the use of a catalyst of 8% $LaCl_3$ on HFZ-33 calcined at (a) 700°C and (b) 900°C.

| Run | $O_2/EDC$ ratio | Contact time (sec) | Tempera-ture (°C) | Mole % conv. EDC | % Yield | |
|---|---|---|---|---|---|---|
| | | | | | VCM | $CO+CO_2$ |
| a | | | | | | |
| 1 | 0.29 | 15.2 | 250 | 29.9 | 56.5 | 39.8 |
| 2 | 0.29 | 13.9 | 300 | 42.5 | 63.8 | 33.3 |
| 3 | 0.20 | 17.2 | 250 | 13.8 | 80.5 | 19.3 |
| 4 | 0.20 | 15.7 | 300 | 36.3 | 71.9 | 26.4 |
| 5 | 0.20 | 26.1 | 300 | 45.6 | 62.1 | 27.4 |
| b | | | | | | |
| 1 | 0.29 | 15.2 | 250 | 2.7 | 56.6 | 40.9 |
| 2 | 0.29 | 13.9 | 300 | 11.5 | 50.6 | 45.2 |
| 3 | 0.20 | 17.2 | 250 | 4.7 | 62.2 | 26.6 |
| 4 | 0.20 | 15.7 | 300 | 8.1 | 48.2 | 46.1 |
| 5 | 0.20 | 26.1 | 300 | 20.3 | 52.7 | 44.1 |

The data shows that the catalyst calcined at 700°C is a more effective catalyst, particularly at higher EDC cracking temperatures.

Example 5

The following data is from runs using a catalyst of 16% $LaCl_3$ on HFZ-55.

| Run | $O_2/EDC$ ratio | Contact time (sec) | Tempera-ture (°C) | Mole % conv. EDC | % Yield | |
|---|---|---|---|---|---|---|
| | | | | | VCM | $CO+CO_2$ |
| 1 | 0.29 | 15.2 | 250 | 22.1 | 71.9 | 24.5 |
| 2 | 0.20 | 17.2 | 250 | 16.8 | 65.1 | 26.1 |
| 3 | 0.20 | 28.6 | 250 | 26.8 | 56.0 | 37.5 |
| 4 | 0.29 | 13.9 | 300 | 27.9 | 52.5 | 42.4 |
| 5 | 0.20 | 26.1 | 300 | 23.8 | 55.9 | 36.6 |
| 6 | 0.20 | 15.7 | 300 | 34.9 | 61.2 | 33.0 |
| 7 | 0.20 | 8.4 | 300 | 18.7 | 73.9 | 22.1 |
| 8 | 0.20 | 5.8 | 300 | 17.0 | 81.8 | 15.2 |

The data shows that higher conversions of EDC were obtained at 300°C than 250°C and that a contact time of about 15 seconds gave the best results.

# EP 0 162 457 B1

**Claims**

1. In a process of dehydrohalogenation of ethylene dichloride to vinyl chloride at elevated temperatures in the presence of oxygen, the improvement which comprises the use as a catalyst of a composition of a rare earth metal chloride deposited on a zeolite.

2. A process of Claim 1 wherein the temperature of the reaction is from about 150°C to about 500°C at a pressure from about 1 to about 20 atmospheres (1—21 kg/cm$^2$; 101,400—2,070,000 Pa).

3. A process of Claim 2 wherein the catalyst contains from about 0.1% to about 30% by weight, based on the total weight of the catalyst of a chloride of the rare earth metal group consisting of Lanthanum, Proseodymium, Neodymium, and mixtures thereof.

4. A process of Claim 3 where the catalyst contains a solid synthetic zeolite having a pore opening of above 0,5 nm (5 Å).

5. A process of Claim 4 wherein the catalyst employed contains Lanthanum chloride at from about 4% to about 16% by weight of the total weight of the catalyst.

6. A process of Claim 1 wherein the oxygen is present in from about 0.05 mole to about 0.5 mole per mole of ethylene dichloride.

7. A process for the production of vinyl chloride monomer comprising (a) the dehydrohalogenation of ethylene dichloride in the presence of (i) from about 0.05 mole to about 0.5 mole of oxygen per mole of ethylene dichloride and (ii) a catalyst comprising from about 2% to about 20% by weight, based on the total weight of the catalyst, of a rare earth metal chloride on a zeolite, at a temperature from about 150°C to about 500°C at a pressure of from about 101,400 to about 2,070,000 Pa and (b) the recovery and recycle of the hydrogen chloride by-product into an oxyhydrochlorination reaction zone.

**Patentansprüche**

1. Verfahren zur Dehydrohalogenierung von Ethylendichlorid zu Vinylchlorid bei höheren Temperaturen in Gegenwart von Sauerstoff, dadurch gekennzeichnet, daß als Katalysator eine Zusammensetzung eines auf einem Zeolith abgeschiedenen Seltenerdmetallchlorids verwendet wird.

2. Verfahren nach Anspruch 1, worin die Temperatur der Reaktion etwa 150°C bis etwa 500°C bei einem Druck von etwa 1 bis 20 Atmosphären (1 bis 21 kg/cm$^2$; 101 400 bis 2 070 000 Pa) beträgt.

3. Verfahren nach Anspruch 2, worin der Katalysator etwa 0,1 bis etwa 30 Gew.-%, bezogen auf das Gesamtgewicht des Katalysators, eines Chlorids eines Metalls der aus Lanthan, Praseodym, Neodym und deren Mischungen bestehenden Seltenerdmetall-Gruppe enthält.

4. Verfahren nach Anspruch 3, worin der Katalysator einen festen synthetischen Zeolith mit einer Porenöffnung oberhalb von 0,5 nm (5 Å) enthält.

5. Verfahren nach Anspruch 4, worin der eingesetzte Katalysator Lanthanchlorid in einer Menge von etwa 4 bis etwa 16 Gew.-%, des Gesamtgewichts des Katalysators enthält.

6. Verfahren nach Anspruch 1, worin der Sauerstoff in einer Menge von etwa 0,05 mol bis etwa 0,5 mol pro 1 mol Ethylendichlorid anwesend ist.

7. Verfahren zur Erzeugung von Vinylchlorid-Monomer, umfassend
(a) die Dehydrohalogenierung von Ethylendichlorid in Gegenwart von
(i) etwa 0,05 mol bis etwa 0,5 mol Sauerstoff pro 1 mol Ethylendichlorid und
(ii) einem Katalysator, der etwa 2 bis etwa 20 Gew.-%, bezogen auf das Gesamtgewicht des Katalysators, eines Seltenerdmetallchlorids auf einem Zeolith
bei einer Temperatur von etwa 150°C bis etwa 500°C bei einem Druck von etwa 101 400 bis etwa 2 070 000 Pa und
(b) die Isolierung und Rückführung des als Nebenprodukt anfallenden Hydrogenchlorids in eine Reaktionszone der Oxyhydrochlorierung.

**Revendications**

1. Dans un procédé de déshydrohalogénation de dichlorure d'éthylène en chlorure de vinyle à des températures élevées en présence d'oxygène, l'amélioration qui consiste à utiliser comme catalyseur un chlorure de métal des terres rares, déposé sur une zéolite.

2. Un procédé de la revendication 1, dans lequel la température de la réaction est d'environ 150°C à environ 500°C à une pression d'environ 1 à environ 20 atmosphères (1—21 kg/cm$^2$; 101400—2 070 000 Pa).

3. Un procédé de la revendication 2, dans lequel le catalyseur contient d'environ 0,1 à environ 30% en poids, basé sur le poids total du catalyseur d'un chlorure du groupe des métaux des terres rares constitué par le lanthane, le proséadyme, le néodyme et leurs mélanges.

4. Un procédé de la revendication 3, où le catalyseur contient une zéolite solide synthétique présentant une ouverture des pores supérieure à 0,5 nm (5 Å).

5. Un procédé de la revendication 4, dans lequel le catalyseur utilisé contient du chlorure de lanthane selon environ 4% à environ 16% en poids du poids total du catalyseur.

6. Un procédé de la revendication 1, dans lequel l'oxygène est présent d'environ 0,05 mole à environ 0,5 mole par mole de dichlorure d'éthylène.

7

7. Un procédé pour la production de chlorure de vinyle monomère comprenant (a) la déshydrohalogénation de dichlorure d'éthylène en présence de (i) d'environ 0,05 mole à environ 0,5 mole d'oxygène par mole de dichlorure d'éthylène et (ii) un catalyseur comprenant d'environ 2% à environ 20% en poids, basé sur le poids total du catalyseur, d'un chlorure d'un métal de terres rares sur un zéolite, à une température d'environ 150°C à environ 500°C sous une pression d'environ 101400 à environ 2 070 000 Pa et (b) la récupération et le recyclage de l'acide chlorhydrique sous-produit dans une zone de réaction d'oxychlorhydratation.